# EUROPEAN PATENT SPECIFICATION

(11) **EP 4 312 948 B1**
(45) Date of publication and mention of the grant of the patent: **25.09.2024**
(21) Application number: 22710035.1
(22) Date of filing: 02.03.2022
(51) Int. Cl.: A61K 8/02, A61K 8/73, A61K 8/46, A61K 8/44, A61Q 19/10, A61Q 5/02

(54) **CLEANSING POWDER COMPOSITION**
REINIGUNGSPULVERZUSAMMENSETZUNG
COMPOSITION DE POUDRE DE NETTOYAGE

(30) Priority: 29.03.2021 EP 21165694
(43) Date of publication of application: 07.02.2024
(73) Proprietor: Unilever IP Holdings B.V., 3013 AL Rotterdam (NL); Unilever Global IP Limited, Wirral, Merseyside CH62 4ZD (GB)
(72) Inventor: CRAIG, Jennifer Lyn, 6708 WH Wageningen (NL); SALIBA, Mariana, 6708 WH Wageningen (NL); POE, Gary, 6708 WH Wageningen (NL)
(74) Representative: Fijnvandraat, Arnoldus
(86) International application number: PCT/EP2022/055232
(87) International publication number: WO 2022/207219

(56) References cited:
- CN-A- 111 297 792
- DATABASE GNPD [online] MINTEL; 4 September 2018 (2018-09-04), ANONYMOUS: "Clear Powder Face Wash", XP055707295, retrieved from https://www.gnpd.com/sinatra/recordpage/5937279/ Database accession no. 5937279
- DATABASE GNPD [online] MINTEL; 13 January 2020 (2020-01-13), ANONYMOUS: "The Powder of Youth No. 1", XP055845814, retrieved from https://www.gnpd.com/sinatra/recordpage/7103577/ Database accession no. 7103577
- DATABASE GNPD [online] MINTEL; 4 September 2018 (2018-09-04), ANONYMOUS: "Clear Powder Face Wash", XP055707295, retrieved from https://www.gnpd.com/sinatra/recordpage/5937279/ Database accession no. 5937279
- DATABASE GNPD [online] MINTEL; 13 January 2020 (2020-01-13), ANONYMOUS: "The Powder of Youth No. 1", XP055845814, retrieved from https://www.gnpd.com/sinatra/recordpage/7103577/ Database accession no. 7103577
- DATABASE GNPD [online] MINTEL; 2 December 2020 (2020-12-02), ANONYMOUS: "Daily Microfoliant Refill", XP055845803, retrieved from https://www.gnpd.com/sinatra/recordpage/8320953/ Database accession no. 8320953
- ANONYMOUS: "Sino Lion USA Product Catalog", 30 June 2020 (2020-06-30), XP055845886, Retrieved from the Internet <URL:https://nysuppliers20.mapyourshow.com/8_0/explore/collateral_redirect.cfm?CollateralID=290&CFID=4153376&CFTOKEN=b70cfb8c9e4e63fa-AE86090F-D75E-C7EB-DDE03BE9FE6CA604> [retrieved on 20210929]

## Description

### Field of the Invention

The present invention is directed to a cleansing powder composition. More particularly, the cleansing powder composition comprises a surfactant system having a taurate and glycinate, a polysaccharide builder and at least one emulsifier having an HLB of at least 8. The cleansing powder composition is gentle during use, generates consumer desirable lathering characteristics and is substantially free of bicarbonate. Such a cleansing powder composition unexpectedly does not aggregate, is convenient to evacuate from packaging and is easy to hydrate for a single use application.

### Background of the Invention

Liquid based cleansing compositions, such as shampoos, body washes and hard surface cleaners are common and enjoyed by many consumers. These conventional compositions typically have water as the predominant ingredient, and they are often sold in plastic bottles, plastic pump containers and tubes. The compositions are conventionally formulated to have a viscosity that is customary for consumer use and easy for evacuation from the packaging they are sold in.

It is often publicized that the world's oceans will soon have more plastic than fish. Given environmental concerns and the desire for consumers and conscious companies to do more for the planet, there is a strong desire to use less plastic when selling products, including consumer products. In view of this, efforts have been made to sell product in concentrate form, and therefore, to ship product that comprises less water. The difficulty with concentrates is consumers often do not like adding additional water to the concentrate and performing further work, like stirring and shaking, to convert the concentrate into an end usable product. As to the hydrated product originating from concentrates, common complaints include that the product is not homogeneous after adding water and/or of undesirable viscosity. Moreover, concentrates are not typically used to prepare single use amounts (or dosages) of end use consumer product.

Coupled with the desire to use less plastic, there is increasing consumer desire to regularly clean, for example, their hands and surfaces to minimize the risk of getting sick. Hand washing is one of the most effective ways to prevent the spread of germs and bacteria. Studies show that hand washing reduces the chances of getting the common cold by about 50% and it deactivates the influenza virus. During a pandemic, consumers know it is in their best interest to consistently wash their hands, faces and any other surfaces they may use or contact.

It is of increasing interest to develop a cleansing powder composition that is easy to carry and hydrate, and that results in consumer desirable lathering with limited shear and water. It is also of increasing interest to have a cleansing powder composition that can be easily used to prepare single use applications of end use consumer wash product. This invention, therefore, is directed to a cleansing powder composition that comprises a surfactant system having a taurate and glycinate, a polysaccharide builder and at least one emulsifier having an HLB of at least 8, as defined in the attached claims. The cleansing powder composition is gentle when using, and substantially free of bicarbonate. Such a cleansing powder composition unexpectedly does not aggregate, is convenient to evacuate from packaging and is easy to hydrate for a single use application.

### Additional Information

Efforts have been disclosed for making powdered compositions. In U.S. Published Patent Application No. 2009/099050 A1, a facial cleansing powder with starch powder and phytic acid is described.

Other efforts have been disclosed for making compositions that can be in the form of a powder.

In U.S. Patent No. 8,232,417, compositions with artemisinin derivatives peptides, amino acids and amino sugars are described.

Even other efforts have been disclosed for making powder compositions. In European Patent No. EP0995423 B1, make-up removing and cleansing powders having starch and 50 to 90% by weight of an oily phase are described.

None of the additional information describes a cleansing powder application as described and claimed in the present application.

### Summary of the Invention

In a first aspect, the present invention is directed to a cleansing powder composition comprising:
a) a surfactant system comprising a taurate and glycinate;
b) a polysaccharide builder; and
c) an emulsifier having an HLB of at least 8.

In a second aspect, the present invention is directed to method of cleaning with the cleansing powder composition of the first aspect of this invention by hydrating the composition with shear to produce an end use wash composition and contacting skin, hair, nails or an inanimate object with the end use wash composition.

The present description thus also discloses the use of a composition comprising:
a) a surfactant system comprising a taurate and glycinate;
b) a polysaccharide builder; and
c) an emulsifier having an HLB of at least 8 to clean skin, hair, nails or an inanimate object.

Cleansing powder composition as used herein means a composition that is hydratable and suitable to clean skin, hair, nails or an inanimate object. Surfactant system comprising a taurate and glycinate means the total source of surfactant in the cleansing composition powder that comprises both a taurate and a glycinate where the total surfactant in the system is at least 70%, and preferably, at least 80%, and most preferably, at least 90% by weight taurate and glycinate. In an embodiment of the invention, taurate and glycinate make up from 92.5 to 100% by weight of the surfactant in the surfactant system. In yet another embodiment of the invention, taurate and glycinate make up 100% by weight of the total surfactant (excluding emulsifier) in the system. Polysaccharide builder means a carbohydrate that builds (i.e., contributes to) the viscosity of the end use wash composition and/or builds smooth sensory benefits. It comprises microcrystalline cellulose, maltodextrin and Oryza sativa (rice) starch at a weight ratio of 35 to 75% microcrystalline cellulose to 3 to 15% maltodextrin to 20 to 60% by weight Oryza sativa (rice) starch, based on total weight of the polysaccharide builder. Skin, as used herein, includes skin on the feet, face, neck, chest, arms (including under arms), hands, legs, buttocks, back and scalp. Inanimate object is meant to include a hard surface like a counter, glass, an appliance door, vehicle and a porcelain fixture such as a dish, mug, sink, tub or toilet. Inanimate object is also meant to include materials comprising nylon, cotton and/or polyester (e.g., clothes, curtains, shirts, rugs, sneakers, upholstery), and fruits and vegetables that are typically washed before consumption. Substantially free of bicarbonate means less than 5.0%, and preferably, less than 2.75%, and most preferably, less than 1.25% by weight bicarbonate based on total weight of the cleansing powder composition. In an embodiment of the invention, the cleansing powder composition comprises no (0.0% by weight) bicarbonate. The cleansing powder composition after hydration (i.e., combining with water) is herein referred to as an end use wash composition and is therefore a precursor to an end use composition. The end use wash composition includes shampoos for hair washing, body washes, automobile wash products as well as home care wash compositions like hard surface and window cleaners, toilet bowl cleaners and laundry detergents. In one embodiment, the end use wash composition of the present invention is a hand, body, face, scalp and/or nail wash composition. In another embodiment, the end use wash composition is a shampoo (i.e., hair washing) composition. In still another embodiment, the end use wash composition is a home care composition. Powder flow, as used herein, means easy to shake out of a shaker, where powder clumping and static cohesion are not visually observed.

Unless explicitly stated otherwise, all ranges described herein are meant to include all ranges subsumed therein. The term comprises is meant to encompass the terms consisting essentially of and consisting of. For the avoidance of doubt, a composition comprising taurate, glycinate, polysaccharide builder and emulsifier is meant to include a composition consisting essentially of the same and a composition consisting of the same. As to the percentages used herein, the same are meant to be by weight of ingredient, (e.g., not including any water it may be supplied with) unless noted otherwise. Except in the operating and comparative examples, or where otherwise explicitly indicated, all numbers used in this description indicating amounts, or ratios of materials and/or use thereof are to be understood as modified by the word "about".

### Detailed Description of the invention

As to the taurate used in the surfactant system of the cleansing powder composition, the same is limited only to the extent that it is one suitable for use in a consumer product. Illustrative examples of the taurate surfactant that may be used in the cleansing powder composition of the invention include, for example, those which are acylamides of taurine or N-methyltaurine, and salts thereof. For example, taurates suitable for use are acyl taurates represented by the general formulae:

R¹C(O)N(R²)(CH₂)_{y}SO₃M (I),

and

R¹C(O)N(R²)CH₂CH₂SO₃M (II),

where R¹ is C₆ to C₃₀, more particularly, C₆ to C₂₄ alkyl, y is 2 or 3, R² is hydrogen or methyl, and M is hydrogen or a solubilizing cation such as, for example, hydrogen, ammonium, alkali metal cation, a lower C₁ to C₄, alkanol ammonium cation and/or a basic amino acid cation. In one embodiment, R¹ is C₈ to C₁₈ alkyl. In another embodiment at least half of the R¹ groups are C₈-C₁₈ alkyl. In still another embodiment at least half of the R¹ groups are C₁₀ to C₁₄ alkyl. R¹ may be saturated or unsaturated. In yet another embodiment R² is methyl.

Illustrative acyl taurates that may be used in the surfactant system of the invention include, for example, taurates commonly known as sodium methyl lauroyl taurate, sodium methyl myristoyl taurate, sodium methyl cocoyl taurate, sodium methyl oleoyl taurate, sodium cocoyl taurate, mixtures thereof or the like. In an embodiment of the invention, the taurate used is sodium methyl lauroyl taurate.

As to the glycinate used in the surfactant system of the cleansing powder composition, the same is limited only to the extent that it is one suitable for use in a consumer product. Illustrative examples of the glycinates and salts thereof often preferred for use include those having the formula:

R³C(O)N(R⁴)CH₂CO₂X (III),

where R³ is a C₈-C₂₄ (preferably a C₁₀-C₂₄) alkyl group, R⁴ is H or a CH₃ and X is hydrogen a cation such as sodium, potassium, ammonium, a triethanolammonium ion or a mixture thereof.

Illustrative examples of the most preferred glycinates suitable for use in the cleansing powder composition of the present invention include sodium cocoyl glycinate, potassium cocoyl glycinate or a mixture thereof.

The surfactant system used in the cleansing powder composition of this invention typically makes up from 12 to 38%, and preferably, from 16 to 32%, and most preferably, from 20 to 30% by weight of the cleansing powder composition. In an embodiment of the invention, the surfactant system makes up from 22 to 28% by weight of the cleansing powder composition.

As to the taurate and glycinate present, typically the combination of taurate and glycinate is at least 50%, and preferably, at least 60%, and most preferably, at least 70% by weight taurate based on total weight of taurate and glycinate in the surfactant system. In an embodiment of the invention, glycinate makes up from 2% to 25% by weight of the total weight of taurate and glycinate in the surfactant system. In another embodiment, glycinate makes up from 3.5% to 12% by weight of the total weight of taurate and glycinate in the surfactant system. In even another embodiment, glycinate makes up from 4% to 8% by weight of the total weight of taurate and glycinate in the surfactant system.

The glycinate used in the cleansing powder composition should not after hydration comprise more that 5% by weight nonionic glycinate, and preferably, not more that 3% by weight nonionic glycinate based on total weight of glycinate in the cleansing powder composition. In an embodiment of the invention, less than 1.5%, and preferably, no (0.0% by weight) glycinate is nonionic (i.e., it is anionic) after the cleansing powder composition is hydrated based on total weight of glycinate used in the cleansing powder composition. Therefore, upon hydration, it is expected that the pH of the cleansing powder composition will be over 5 to about 8, and preferably, from 5.4 to 7.5, and most preferably, from 6.2 to 7.2 where pH is determined using a Thermo Fisher Scientific pH meter.

While not required, additional conventional surfactants found in wash compositions can be included in the surfactant system used in the cleansing powder composition of this invention. Such surfactants may optionally include amphoteric surfactants like sodium lauroamphoacetate, sodium cocoamphoacetate, potassium lauroamphoacetate, potassium cocoamphoacetate or mixtures thereof. Zwitterionic surfactants may optionally be used and these include betaines such as lauryl betaine, betaine citrate, cocodimethyl carboxymethyl betaine, cocoamidopropyl betaine, cocoalkyldimethyl betaine, and laurylamidopropyl betaine. Still other zwitterionic surfactants suitable for use include a cocoamidopropyl sultaine, cocamidopropyl hydroxysultaine or a mixture thereof. Optionally, cationic surfactants may be included in the wash composition of the present invention. Those that may be used includes heterocyclic ammonium salts such as cetyl or stearyl pyridinium chloride, alkyl amidoethyl pyrrylinodium methyl sulfate, lapyrium chloride or a mixture thereof. Additional surfactants that may optionally be used in the surfactant system of the present invention include an anionic like sodium lauroyl glutamate, sodium cocoyl glutamate, sodium lauroyl isethionate, sodium cocoyl isethionate or a mixture thereof. In an embodiment of the present invention, less than 5% by weight of the total weight of surfactant system is isethionate. In another embodiment of the present invention, less than 2.5% by weight of the total weight of the surfactant system is isethionate, and preferably, the surfactant system is free of (0.0% by weight) isethionate. Nonionic surfactants that may optionally be used include polyglycerol esters like polyglyceryl-8 caprylate, polyglycerol-8 caprate, polyglyceryl-8 myristate, polyglyceryl-8 palmitate, polyglyceryl-9 caprylate, polyglycerol-9 caprate, polyglyceryl-9 laurate, polyglyceryl-9 myristate, polyglyceryl-9 palmitate or a mixture thereof. As noted, however, the total surfactant system is at least 70%, and preferably, at least 80%, and most preferably, at least 90% by weight taurate and glycinate. In an embodiment of the invention, taurate and glycinate make up from 92.5 to 100% by weight of the surfactant in the surfactant system. In yet another embodiment of the invention, taurate and glycinate make up 100% by weight of the total surfactant in the system.

Polysaccharide builder used in the present invention is limited only to the extent that the same is suitable for use in a topical composition. These includes include fibers, starches, gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel ^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

In an embodiment of the invention, a polysaccharide builder used is rice starch and/or a corn starch referred to as INCI: Zea Mays (Corn) Starch like, for example, the product sold under the commercial name Farmal^{™} CS 3650 by Ingredion. In another embodiment a polysaccharide builder used is unmodified rice starch referred to as INCI: Oryza sativa (rice) starch that is also made commercially available from Ingredion under the Nativacare^{™} name. In still another embodiment a polysaccharide builder used is maltodextrin made available as Farmal^{™} MD 10 by Ingredion (i.e., equivalent Dextrose (DE) 9.0 to 12.0). In even another embodiment, the polysaccharide builder used is microcrystalline cellulose made available by Active Organics under the name Acticel^{®} 12 name (CAS 9004-34-6).

Synthetic polymers (non-polysaccharide) may optionally be used as builders in addition to the polysaccharide builders. This category includes crosslinked polyacrylates such as the Carbomers, polyacrylamides such as Sepigel 305 and taurate copolymers such as Simulgel EG and Arlstoflex AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium bicarbonate, fumed silica, and magnesium-aluminum-silicate may also optionally be used; however, the cleansing powder composition is preferably substantially free of bicarbonate. As to total builder (i.e, total polysaccharide and non-polysaccharide builder) used in the cleansing powder composition of this invention, no more than 20% by weight, and preferably, no more than 8% by weight, and most preferably, no more than 3% by weight non-polysaccharide builder is used based on total weight of builder in the cleansing powder composition. When optionally used, non-polysaccharide builder can be present in amounts as low as 0.001 % by weight based on total weight of builder in the cleansing powder composition. In an embodiment of the invention, no non-polysaccharide builder is used (0.0% by weight); therefore, the only builder in the cleansing powder composition is polysaccharide builder.

The polysaccharide builder used comprises microcrystalline cellulose, maltodextrin and Oryza sativa (rice) starch at a weight ratio of 35 to 75% microcrystalline cellulose to 3 to 15% maltodextrin to 20 to 60% by weight Oryza sativa (rice) starch, and preferably, a weight ratio of 40 to 70% microcrystalline cellulose to 4 to 12% maltodextrin to 25 to 55% by weight Oryza sativa (rice) starch, and most preferably, a weight ratio of 45 to 65% microcrystalline cellulose to 5 to 10% maltodextrin to 35 to 50% by weight Oryza sativa (rice) starch based on total weight of the polysaccharide builder. In another preferred embodiment, the polysaccharide builder consists essentially of, and most preferably, consists of microcrystalline cellulose, maltodextrin and Oryza sativa (rice) starch.

The cleansing powder composition preferably comprises from 55 to 80%, and preferably, from 57.5 to 77.5%, and most preferably, from 58 to 70% by weight total builder based on total weight of the cleansing powder composition. In an embodiment of the invention, the cleansing powder composition comprises from 61.5 to 68.5% by weight builder based on total weight of the cleansing powder composition.

The polysaccharide builder can be present either as generally round or irregular shape particles. Such particles are also desirably hydrophilic. In an embodiment of the invention, the polysaccharide builder may have a degree of polymerization between 175 and 3000. In still another embodiment, the particles may have an average particle size from 0.5 to 250 microns, and preferably from 5 to 200 microns, and most preferably, from 10 to 175 microns. Average particle size as used herein means the volume-mean particle size that refers to the diameter of the particle in the aqueous dispersion. For polymer particles that are not spherical, the diameter of the particle is the average of the long and short axes of the particle. Particle sizes can be measured on a Beckman-Coulter LS 13 320 laser-diffraction particle size analyzer, with an art recognized sieve or any other art recognized device. In an embodiment of the invention, the microcrystalline cellulose has an average particle size that is 3 to 10, and preferably, 4 to 9, and most preferably, 5 to 8 times smaller than the average particle size of the remainder of the total polysaccharide builder used in the cleansing powder composition.

The emulsifier suitable for use in the cleansing powder composition has an HLB of at least 8 where HLB is the balance between hydrophilic and lipophilic parts of amphiphilic molecules. Illustrative emulsifiers that may be used in the compositions of this invention include polyethylene lauryl ether (Brij^{®} 30), polyoxyethylene lauryl ether (Brij^{®} 35), lecithin, methyl cellulose (Methocel^{™}), polyoxyethylene lauryl ether, polyoxyethylene monostearate (Myrj^{™} 45), triethanolamine oleate, polyoxyethylene alkyl phenol (Igepal^{®}), polyethylene glycol 400 monolaurate, polyoxyethylene sorbitan mono-oleate (Tween^{®} 80), polyoxyethylene sorbitan monolaurate (Tween^{®} 20), Polysorbate 60, PEG 8 laurate, Oleth 10, almond glyceride, sodium oleate, potassium oleate or a mixture thereof.

In an embodiment of the invention, the emulsifier used has an HLB from 10 to 20. In another embodiment, the emulsifier has an HLB from 12 to 18, and preferably, from 13.5 to 17.5. In still another embodiment of the invention, emulsifier that is liquid is sprayed on the cleansing powder composition ingredients to avoid any particle clumping or agglomeration. In still another embodiment, the emulsifier used in the cleansing powder composition is a polysorbate, and preferably, Tween^{®} 20.

The amount of emulsifier used in the cleansing powder composition is typically from 1 to 4%, and preferably, from 1.5 to 3.5%, and most preferably, from 1.75 to 2.75% by weight based on total weight of the cleansing powder composition. In an embodiment of the invention, the amount of emulsifier is from 2.0 to 2.5% by weight of the total weight of the cleansing powder composition.

When using the cleansing powder composition of the present invention, powder and water, in no particular order, can placed in a mixing vessel and shaken, stirred and/or agitated with moderate shear. The resulting end use wash composition can be used as desired (e.g., on hands, face and/or hair). The amount of end use wash composition made is determined by consumer preference. For a single use hand, face or hair wash, typically from 1.0 to 12 grams, and preferably, from 1.5 to 10 grams, and most preferably, from 2 to 8 grams of cleansing powder composition is used. The amount of water used with the cleansing powder composition to make end use composition is also determined by consumer preference. Often, the amount/weight of water used with cleansing powder composition to produce end use wash composition is from 0.25 to 4 times, and preferably, from 0.5 to 3 times, and most preferably, from 1 to 2 times the amount/weight of cleansing powder composition used. In an embodiment of the invention, the weight ratio of water to cleansing powder composition used to make end use composition is from 45:55 to 55:45.

In an embodiment of the invention, cleansing powder composition and water, in no particular order, are placed in the hand. Mixing and shearing with both hands results in hand washing and end use wash composition generation simultaneously. End use wash composition for hair washing can be produced in the hands as well. In an embodiment of the invention, water and cleansing composition can be placed in a hand and subsequently supplied to the face or head for face washing or hair and scalp washing where the washing motion provides the shear to make the face wash or shampoo composition as the case may be. In still another embodiment, cleansing powder composition may be applied dry to a substrate like hair, a rug or upholstery that is dry or wet, followed by water (if necessary in the case of prewetted substrate) whereby shear from the hand or an object like a scrub brush results in cleaning and simultaneous generation of cleansing wash composition.

Optional ingredients that may be used in the cleansing powder composition of the present invention include preservatives to assist against the growth of potentially harmful microorganisms when the end use wash composition is made. Suitable traditional preservatives that may be used in the compositions of this invention include alkyl esters of para-hydroxybenzoic acid. Other preservatives include hydantoin derivatives, propionate salts, and a variety of quaternary ammonium compounds. Often preferred preservatives are sodium benzoate, potassium sorbate, iodopropynyl butyl carbamate, phenoxyethanol, methyl paraben, wasabi based preservatives, propyl paraben, imidazolidinyl urea, sodium dehydroacetate and benzyl alcohol. Especially preferred additives suitable to be employed in the cleansing powder composition of the present invention are 1,2-alkanediols like 1,2-octanediol, 1,2 hexanediol or mixtures thereof.

Traditional fragrance components like eugenol, coumarin, linalyl acetate, citronellal, iris concentrate, terpinyl acetate, terpineol, thymol, pinenes (e.g., alpha and beta pinene) and citronellol may optionally be added to the cleansing powder composition as well.

If employed, the traditional preservatives, vicinal diol and/or fragrance component will not make up more than 2%, and preferably, not more than 1%, and most preferably, from 0.2 to 0.85% by weight of the end use wash composition of the present invention. In an embodiment of this invention from 0.2 to 0.8% by weight optional preservative, vicinal diol and/or fragrance component is used, based on total weight of the end use wash composition. In an embodiment of the invention, no traditional preservative, vicinal diol and/or fragrance component (except for what may be provided in the fragrance used in the end use wash composition) is used in the end use wash composition since such a wash composition can be made and used on demand if desired giving the consumer the option to have a preservative free wash product.

Fragrances, fixatives and abrasives may optionally be used in the cleansing powder composition of the present invention. Other optional ingredients suitable for use include zinc pyrithione, octopirox or a mixture thereof, especially when the end use wash composition is shampoo that provides antidandruff benefits. Each of these substances may range from 0.05 to 3%, preferably between 0.1 and 2% by weight of the total weight of the end use wash composition.

Additional optional ingredients that may be used include sensory oils and/or exfoliants. Suitable oils include rose, lime, coconut, lavender oil(s) or mixtures thereof. Illustrative exfoliants suitable for use include salt, sugar, apricot, walnut shell, rice, nutmeg and/or oatmeal powder(s). When used, sensory oils and exfoliants can make up from 0.1 to 2% by weight of the end use wash composition, with the proviso that the total amount of fragrance and sensory oil does not exceed 2.5% by weight of the end use composition, and preferably, not more than 2.0 percent by weight of the composition.

The cosmetic powder composition of the present invention may include vitamins. Illustrative vitamins are Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present may range from 0.001 to 4%, and preferably from 0.01% to 3.5%, optimally from 0.1 to 2.5% by weight of the cosmetic powder composition.

Other optional additives suitable for use in this invention include resorcinols like 4-ethyl resorcinol, 4-hexyl resorcinol, 4-phenylethyl resorcinol, dimethoxytoluyl propyl resorcinol, 4-cyclopentyl resorcinol, 4-cyclohexylresorcinol, alpha- and/or beta-hydroxyacids, petroselinic acid, conjugated linoleic acid, 12-hydroxystearic acid, mixtures thereof or the like. Still other optional additives like ethanol, quaternary ammonium compounds (like cetrimonium chloride, benzalkonium chloride or the like) and may also be included. Such additives, when used, collectively make up from 0.001 to 3%, and preferably, from 0.01 to 2%, and most preferably, from 0.1 to 1.5% by weight of the end use wash composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Salicylic acid is representative of the beta-hydroxycarboxylic acids. Amounts of these materials when present may range from 0.01 to 3%, and preferably, from 0.1 to 2% by weight of the end use wash composition.

A variety of herbal extracts may optionally be included in the cosmetic wash compositions of this invention. Illustrative extracts include those removed from green tea, yarrow, ginsing, marigold, hibiscus, ginko biloba, chamomile, licorice, aloe vera, grape seed, citrus unshiu, willow bark, sage and rosemary. Humectants like glycerol and other polyols may also be included.

Humectants and/or extracts, when used, typically make up from 0.01 to 0.2, and preferably, from 0.01 to 0.15, and most preferably, from 0.02 to 0.1% by weight of the end use composition.

Also optionally suitable for use include materials like chelators (e.g., EDTA), opacifiers (like TiO₂, particle size from 50 to 1200 nm, and preferably, 50 to 350 nm), kaolin, bentonite, zinc oxide, iron oxide, mica, C₈₋₂₂ fatty acid substituted saccharides, lipoic acid, retinoxytrimethylsilane (available from Clariant Corp. under the Silcare 1M-75 trademark), dehydroepiandrosterone (DHEA) or mixtures thereof. Ceramides (including Ceramide 1, Ceramide 3, Ceramide 3B and Ceramide 6) as well as pseudoceramides may also be optionally included as can 10 and/or 12-hydroxystearic acid. Amounts of extract, polyol and these additional materials when used may range from 0.0001 to 3%, and preferably, from 0.001 to 2%, and most preferably, from 0.001 to 1.5% by weight of the end use wash composition.

Anticaking agents like tricalcium phosphate may optionally be used in the cleansing powder composition. When used, anticaking agents make up from 0.01 to 3.5%, and preferably, from 0.5 to 3%, and most preferably, from 1 to 3% by weight of the cleansing powder composition. Conditioning agents like hydroxypropyltrimonium chloride, 5-ureidohydantoin and/or glyoxyldiureide may be used. The components when used make up from 0.5 to 4%, and preferably, from 0.75 to 4%, and most preferably, from 1 to 3% by weight of the cleansing powder composition.

Occlusives like almond, olive, safflower, and mineral oil may also be used in the cleansing powder composition of the present invention. When used, these materials make up less than 0.06%, and preferably, less than 0.03% by weight of the cleansing powder composition.

Sunscreen actives may also be optionally included in cosmetic powder composition of the present invention. Particularly preferred are such materials as ethylhexyl p-methoxycinnamate, available as Parsol MCX^{®}, Avobenzene, available as Parsol 1789^{®} and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.01 to 3%, preferably from 0.5 to 2%, optimally from 0.75 to 1.5% by weight of the end use wash composition.

Conventional buffers/pH modifiers may be used. These include commonly employed additives like sodium hydroxide, potassium hydroxide, hydrochloric acid, citric acid, triethanolamine, citrate/citric acid buffers or mixtures thereof. These materials are added at amounts to obtain the desired pH of the end use wash composition.

In an embodiment of the invention, the end use wash composition of the present invention is preferably free of sulfate, and therefore, having less than 2.5% and preferably, less than 1.5%, and most preferably, less than 0.5% by weight of a sulfate. In an especially preferred embodiment, the end use wash composition has no (0.0% by weight) sulfate comprising component. In still another preferred embodiment, less than 5%, and preferably, less than 3%, and most preferably, less than 1.5% by weight of the end use wash composition comprises water insoluble ingredients. In a most preferred embodiment, the end use wash composition of the present invention has no (0.0% by weight) water insoluble ingredient. In another preferred embodiment, no ingredient is used in the cosmetic powder composition that will enhance and cause clumping or agglomerating of the powder composition.

The bulk density of the cosmetic powder composition is typically from 0.2 to 1.2, and preferably, from 0.3 to 0.9, and most preferably, from 0.35 to 0.7 g/cm³ where bulk density is measured by art recognized techniques that use, for example, a gas pycnometer made commercially available by Microtrac MRB.

The viscosity of the end use wash composition of this invention is typically from 100 to 8,000 cps, and preferably, from 120 to 7,000 cps, and most preferably, from 200 to 6000 cps. Viscosity may be measured with art recognized instrumentation such as a Brookfield Viscometer RV5, Model D220, using a RV spindle 5 at 20 RPM, 60 seconds at 25°C where the end use wash composition was prepared with a 50:50 mixture of water and cleansing powder composition.

When preparing cleansing powder composition, ingredients may be mixed and/or agitated at atmospheric pressure. The temperature at which mixing and agitation occurs is typically from 20 to 50°C. Mixing and/or agitation is stopped when a homogeneous mixture is obtained and the resulting cleansing powder composition is free of aggregated particles. The particles of the cleansing powder composition will have a size from 0.2 to 600 microns, and preferably, from 0.3 to 500 microns, and most preferably, from 0.4 to 350 microns as determined with art recognized devices as noted herein.

A wide variety of packaging can be employed to store the cleansing powder composition of this invention. Jars, sachets, shakers, bags, pumps, bottles, metallic containers as well as plastic containers may be used. Preferably, the packaging used with the cleansing powder composition of the present invention is packaging made from recycled material like post-consumer resins or biodegradable material. Most preferably, the cleansing powder composition is sold in biodegradable packaging material, like paper or cardboard material, whereby the jar or bottle, as the case may be, is refilled with cleansing powder composition sold in biodegradable packaging. In an especially preferred embodiment, the cleansing powder composition is used by the consumer directly from the biodegradable packaging.

The following examples are provided to facilitate an understanding of the present invention. The examples are not intended to limit the scope of the claims.

### Example I

Cleansing powder compositions were made according to the present invention with the ingredients and amounts set forth in Table I. The ingredients were combined via agitation at atmospheric pressure and at 25°C. Surprisingly, no visual clumping or agglomeration of composition was observed even after the product was stored at 37, 45, and 50°C for twelve (12) weeks in a sealed package. Additionally, the cleansing powder compositions had a bulk density of about 0.43 g/cm³ and was easily flowable in the absence of visual static cohesion.

**TABLE I**

| **Ingredient Name** | **Percent by Weight** |
|---|---|
| Microcrystalline Cellulose | 35 |
| Maltodexrin | 5 |
| *Oryza Sativa (Rice) Starch* | 25 |
| Sodium Methyl Lauroyl Taurate | 20 |
| Potassium Cocoyl Glycinate | 5 |
| Conditioning agent | 2 |
| Niacinamide | 2 |
| Chelator | 0.1 |
| Preservative | 0.9 |
| Occlusive | 0.01 |
| Polysorbate 20 | 2 |
| Fragrance | 0.8 |
| Tricalcium Phosphate | balance |
| | 100.000 |

About four (4) grams of cleansing powder composition described in Table I were applied to the left forearm of trained panelists followed by about four (4) grams of water. The panelists, using moderate shear with their right hands were able to mix the water and cleansing powder composition to generate an end use wash composition that was not grainy and that provided consumer acceptable (creamy) lathering when being used. The flowability of the powder made it easy to remove from a shaker and a biodegradable sachet or pouch.

### Example II

The ingredients of the formulae made in this Example were mixed in a manner similar to the one described in Example I. None of the formulae made in Tables II to V below were made with ingredients consistent with those of the claimed invention. Visual clumping and agglomeration of composition were observed after the product was stored at 37, 45, and 50°C for two (2) weeks in a sealed package. The formulations not made according to this invention were not usable.

**Table II**

| **Ingredient Name** | **Percent by Weight** |
|---|---|
| Sorbitol | 68 |
| Sodium Cocoyl Glycinate | 10 |
| Sodium Lauroyl Glutamate | 3 |
| Oryza Sativa (Rice) Starch | 10 |
| Maltodextrin | 5 |
| Conditioning agent | 2 |
| Chelator | 0.1 |
| Preservative | 1 |
| Tricalcium Phosphate | Balance |
| | 100 |

**TABLE III**

| **Ingredient Name** | **Percent by Weight** |
|---|---|
| Sorbitol | 65 |
| Sodium Cocoyl Glycinate | 10 |
| Oryza Sativa (Rice) Starch | 10 |
| Maltodextrin | 5 |
| Lactose | 5 |
| Conditioning agent | 2 |
| Chelator | 0.1 |
| Preservative | 1 |
| Tricalcium Phosphate | Balance |
| | 100 |

**TABLE IV**

| **Ingredient Name** | **Percent by Weight** |
|---|---|
| Sorbitol | 70 |
| Sodium Cocoyl Glycinate | 10 |
| Oryza Sativa (Rice) Starch | 10 |
| Maltodextrin | 5 |
| Conditioning agent | 2 |
| Chelator | 0.1 |
| Preservative | 1 |
| Tricalcium Phosphate | Balance |

**TABLE V**

| **Ingredient Name** | **Percent by Weight** |
|---|---|
| Oryza Sativa (Rice) Starch | 80 |
| Sodium Cocoyl Isethionate | 10 |
| Maltodextrin | 5 |
| Conditioning agent | 2 |
| Chelator | 0.1 |
| Preservative | 1 |
| Tricalcium Phosphate | Balance |
| | 100 |

## Claims

1. A cleansing powder composition comprising:
a) a surfactant system comprising a taurate and glycinate;
b) a polysaccharide builder; and
c) an emulsifier having an HLB of at least 8,
wherein the total surfactant in the system is at least 70% by weight taurate and glycinate, and further wherein the polysaccharide builder comprises microcrystalline cellulose, maltodextrin and Oryza sativa (rice) starch at a weight ratio of 35 to 75% microcrystalline cellulose to 3 to 15% maltodextrin to 20 to 60% by weight Oryza sativa (rice) starch, based on total weight of the polysaccharide builder.

2. The cleansing powder composition according to claim 1 wherein the taurate is sodium methyl lauroyl taurate, sodium methyl myristoyl taurate, sodium methyl cocoyl taurate, sodium methyl oleoyl taurate, sodium lauroyl taurate, sodium cocoyl taurate, or a mixture thereof and the glycinate is sodium cocoyl glycinate, potassium cocoyl glycinate, sodium lauroyl glycinate, potassium lauroyl glycinate or a mixture thereof.

3. The cleansing powder composition according to claims 1 or 2 wherein the total surfactant in the system is at least 80%, and preferably, at least 90% by weight taurate and glycinate.

4. The cleansing powder composition according to any of the preceding claims wherein the surfactant system is at least 50%, and preferably, at least 60%, and most preferably, at least 70% by weight taurate based on total weight of taurate and glycinate in the surfactant system.

5. The cleansing powder composition according to any of the preceding claims wherein glycinate makes up from 2% to 25%, and preferably, 3.5% to 12%, and most preferably, 4% to 8% by weight of total weight of taurate and glycinate in the surfactant system.

6. The cleansing powder composition according any of the preceding claims wherein the composition is a precursor to a hands, face, body, nails or inanimate object wash composition.

7. The cleansing powder composition according any of the preceding claims wherein the composition further comprises an emulsifier having an HLB from 10 to 20, and preferably, from 12 to 18.

8. The cleansing powder composition according any of the preceding claims wherein the surfactant system used makes up from 12 to 38%, and preferably, from 16 to 32%, and most preferably, from 20 to 30% by weight of the cleansing powder composition, and even more preferably, 22 to 28% by weight of the cleansing powder composition.

9. The cleansing powder composition according to any of the preceding claims wherein the polysaccharide builder comprises microcrystalline cellulose, maltodextrin and Oryza sativa (rice) starch at a weight ratio of 40 to 70% microcrystalline cellulose to 4 to 12% maltodextrin to 25 to 55% by weight Oryza sativa (rice) starch, and preferably, a weight ratio of 45 to 65% microcrystalline cellulose to 5 to 10% maltodextrin to 35 to 50% by weight Oryza sativa (rice) starch, based on total weight of the polysaccharide builder.

10. The cleansing powder composition according to any of the preceding claims wherein the emulsifier has an HLB from 10 to 20, and preferably, from 12 to 18, and most preferably, from 13.5 to 17.5.

11. The cleansing powder composition according to any of the preceding claims wherein the composition comprises from 55 to 80%, and preferably, from 57.5 to 77.5%, and most preferably, from 58 to 70% by weight total builder based on total weight of the cleansing powder composition.

12. A wash composition comprising the cleansing powder composition according to any of the preceding claims hydrated with water.

13. The wash composition according to claim 12 wherein water is present at a weight that is from 0.25 to 4 times cleansing powder composition weight used to make the wash composition.

14. The wash composition according to claims 12 and 13 wherein the wash composition is a composition for skin, hair, nails or an inanimate object.

15. A method for washing comprising the steps of:
a) hydrating the cleansing powder composition according to claims 1 to 11 to produce a wash composition; and
b) contacting skin, hair, nails or an inanimate object with the wash composition
wherein the cleansing powder composition is hydrated with shear to produce wash composition before or after contact with the skin, hair, nails or an inanimate object.

## Patentansprüche

1. Reinigungspulverzusammensetzung, umfassend:
a) ein Tensidsystem, umfassend ein Taurat und ein Glycinat;
b) einen Polysaccharid-Builder; und
c) einen Emulgator mit einem HLB-Wert von mindestens 8,
wobei das Gesamttensid in dem System zu mindestens 70 Gewichts-% aus Taurat und Glycinat besteht und wobei ferner der Polysaccharid-Builder mikrokristalline Cellulose, Maltodextrin und *Oryza sativa* (Reis)-Stärke in einem Gewichtsverhältnis von 35 bis 75 Gewichts-% mikrokristalline Cellulose zu 3 bis 15 Gewichts-% Maltodextrin zu 20 bis 60 Gewichts-% *Oryza sativa* (Reis)-Stärke, bezogen auf das Gesamtgewicht des Polysaccharid-Builders, umfasst.

2. Reinigungspulverzusammensetzung nach Anspruch 1, wobei das Taurat Natriummethyllauroyltaurat, Natriummethylmyristoyltaurat, Natriummethylcocoyltaurat, Natriummethyloleoyltaurat, Natriumlauroyltaurat, Natriumcocoyltaurat oder eine Mischung davon ist und das Glycinat Natriumcocoylglycinat, Kaliumcocoylglycinat, Natriumlauroylglycinat, Kaliumlauroylglycinat oder eine Mischung davon ist.

3. Reinigungspulverzusammensetzung nach den Ansprüchen 1 oder 2, wobei das Gesamttensid in dem System zu mindestens 80 Gewichts-% und bevorzugt zu mindestens 90 Gewichts-% aus Taurat und Glycinat besteht.

4. Reinigungspulverzusammensetzung nach einem der vorhergehenden Ansprüche, wobei das Tensidsystem zu mindestens 50 Gewichts-% und bevorzugt zu mindestens 60 Gewichts-% und höchst bevorzugt zu mindestens 70 Gewichts-% aus Taurat, bezogen auf das Gesamtgewicht von Taurat und Glycinat in dem Tensidsystem, besteht.

5. Reinigungspulverzusammensetzung nach einem der vorhergehenden Ansprüche, wobei das Glycinat 2 bis 25 Gewichts-% und bevorzugt 3,5 bis 12 Gewichts-% und höchst bevorzugt 4 bis 8 Gewichts-% des Gesamtgewichts von Taurat und Glycinat in dem Tensidsystem ausmacht.

6. Reinigungspulverzusammensetzung nach einem der vorhergehenden Ansprüche, wobei die Zusammensetzung ein Vorläufer einer Waschzusammensetzung für Hände, Gesicht, Körper, Nägel und unbelebte Gegenstände ist.

7. Reinigungspulverzusammensetzung nach einem der vorhergehenden Ansprüche, wobei die Zusammensetzung außerdem einen Emulgator mit einem HLB-Wert von 10 bis 20 und bevorzugt von 12 bis 18 umfasst.

8. Reinigungspulverzusammensetzung nach einem der vorhergehenden Ansprüche, wobei das verwendete Tensidsystem 12 bis 38 Gewichts-% und bevorzugt 16 bis 32 Gewichts-% und höchst bevorzugt 20 bis 30 Gewichts-% der Reinigungspulverzusammensetzung und noch bevorzugter 22 bis 28 Gewichts-% der Reinigungspulverzusammensetzung ausmacht.

9. Reinigungspulverzusammensetzung nach einem der vorhergehenden Ansprüche, wobei der Polysacharid-Builder mikrokristalline Cellulose, Maltodextrin und *Oryza sativa* (Reis)-Stärke in einem Gewichtsverhältnis von 40 bis 70 Gewichts-% mikrokristalline Cellulose zu 4 bis 12 Gewichts-% Maltodextrin zu 25 bis 55 Gewichts-% *Oryza sativa* (Reis)-Stärke umfasst und bevorzugt in einem Gewichtsverhältnis von 45 bis 65 Gewichts-% mikrokristalline Cellulose zu 5 bis 10 Gewichts-% Maltodextrin zu 35 bis 50 Gewichts-% *Oryza sativa* (Reis)-Stärke, bezogen auf das Gesamtgewicht des Polysaccharid-Builders, umfasst.

10. Reinigungspulverzusammensetzung nach einem der vorhergehenden Ansprüche, wobei der Emulgator einen HLB-Wert von 10 bis 20 und bevorzugt von 12 bis 18 und höchst bevorzugt von 13,5 bis 17,5 aufweist.

11. Reinigungspulverzusammensetzung nach einem der vorhergehenden Ansprüche, wobei die Zusammensetzung 55 bis 80 Gewichts-% und bevorzugt 57,5 bis 77,5 und höchst bevorzugt 58 bis 70 Gewichts-% Gesamt-Builder, bezogen auf das Gesamtgewicht der Reinigungspulverzusammensetzung, umfasst.

12. Waschzusammensetzung, umfassend die mit Wasser hydratisierte Reinigungspulverzusammensetzung nach einem der vorhergehenden Ansprüche.

13. Waschzusammensetzung nach Anspruch 12, wobei das Wasser mit einem Gewicht vorliegt, das dem 0,25- bis 4-fachen des Gewichts der zur Herstellung der Waschzusammensetzung verwendeten Reinigungspulverzusammensetzung entspricht.

14. Waschzusammensetzung nach den Ansprüchen 12 und 13, wobei die Waschzusammensetzung eine Zusammensetzung für Haut, Haare, Nägel oder ein unbelebtes Objekt ist.

15. Waschverfahren, umfassend die Schritte:
a) Hydratisieren der Reinigungspulverzusammensetzung nach den Ansprüchen 1 bis 11, um eine Waschzusammensetzung herzustellen; und
b) Inkontaktbringen von Haut, Haar, Nägeln oder einem unbelebten Objekt mit der Waschzusammensetzung, wobei die
Reinigungspulverzusammensetzung unter Scherung hydratisiert wird, um eine Waschzusammensetzung vor oder nach dem Kontakt mit der Haut, dem Haar, den Nägeln oder einem unbelebten Objekt herzustellen.

## Revendications

1. Composition nettoyante en poudre comprenant :
a) un système tensioactif comprenant un taurate et un glycinate ;
b) un adjuvant polysaccharidique ; et
c) un émulsifiant ayant un indice HLB d'au moins 8,
dans laquelle le tensioactif total dans le système est constitué à 70 % en poids de taurate et glycinate, et en outre dans laquelle l'adjuvant polysaccharidique comprend de la cellulose microcristalline, de la maltodextrine et de l'amidon d'Oryza sativa (riz) en un rapport en poids de 35 à 75 % en poids de cellulose microcristalline pour 3 à 15 % de maltodextrine pour 20 à 60 % en poids d'amidon d'Oryza sativa (riz), par rapport au poids total de l'adjuvant polysaccharidique.

2. Composition nettoyante en poudre selon la revendication 1, dans laquelle le taurate est le méthyl-lauroyl-taurate de sodium, le méthyl-myristoyl-taurate de sodium, le méthyl-(coprah-yl)taurate de sodium, le méthyl-oléoyl-taurate de sodium, le lauroyl-taurate de sodium, le (coprah-yl)taurate de sodium), ou un mélange de ceux-ci, et le glycinate est le (coprah-yl)glycinate de sodium, le (coprah-yl)glycinate de potassium, le lauroyl-glycinate de sodium, le lauroyl-glycinate de potassium, ou un mélange de ceux-ci.

3. Composition nettoyante en poudre selon la revendication 1 ou 2, dans laquelle le tensioactif total dans le système est constitué à au moins 80 % et de préférence au moins 90 % en poids de taurate et glycinate.

4. Composition nettoyante en poudre selon l'une quelconque des revendications précédentes, dans laquelle le système tensioactif est constitué à au moins 50 %, de préférence au moins 60 %, tout spécialement au moins 70 % en poids de taurate par rapport au poids total de taurate et glycinate dans le système tensioactif.

5. Composition nettoyante en poudre selon l'une quelconque des revendications précédentes, dans laquelle la glycine constitue de 2 % à 25 %, de préférence de 3,5 % à 12 %, tout spécialement de 4 % à 8 % en poids du poids total de taurate et glycinate dans le système tensioactif.

6. Composition nettoyante en poudre selon l'une quelconque des revendications précédentes, laquelle composition est précurseur d'une composition lavante pour les mains, le visage, le corps, les ongles ou les objets inanimés.

7. Composition nettoyante en poudre selon l'une quelconque des revendications précédentes, laquelle composition comprend en outre un émulsifiant ayant un indice HLB de 10 à 20, de préférence de 12 à 18.

8. Composition nettoyante en poudre selon l'une quelconque des revendications précédentes, dans laquelle le système tensioactif utilisé constitue de 12 à 38 %, de préférence de 16 à 32 %, tout spécialement de 20 à 30 % en poids de la composition nettoyante en poudre, plus particulièrement de 22 à 28 % en poids de la composition nettoyante en poudre.

9. Composition nettoyante en poudre selon l'une quelconque des revendications précédentes, dans laquelle l'adjuvant polysaccharidique comprend de la cellulose microcristalline, de la maltodextrine et de l'amidon d'Oryza sativa (riz) en un rapport en poids de 40 à 70 % de cellulose microcristalline pour 4 à 12 % de maltodextrine pour 25 à 55 % en poids d'amidon d'Oryza sativa (riz), et de préférence un rapport en poids de 45 à 65 % de cellulose microcristalline pour 5 à 10 % de maltodextrine pour 35 à 50 % en poids d'amidon d'Oryza sativa (riz), par rapport au poids total de l'adjuvant polysaccharidique.

10. Composition nettoyante en poudre selon l'une quelconque des revendications précédentes, dans laquelle l'émulsifiant a un indice HLB de 10 à 20, de préférence de 12 à 18, tout spécialement de 13,5 à 17,5.

11. Composition nettoyante en poudre selon l'une quelconque des revendications précédentes, laquelle composition comprend de 55 à 80 %, de préférence de 57,5 à 77,5 %, tout spécialement de 58 à 70 % en poids d'adjuvant total par rapport au poids total de la composition nettoyante en poudre.

12. Composition lavante comprenant la composition nettoyante en poudre de l'une quelconque des revendications précédentes hydratée avec de l'eau.

13. Composition lavante selon la revendication 12, dans laquelle de l'eau est présente en un poids qui est de 0,25 à 4 fois le poids de la composition nettoyante en poudre utilisée pour préparer la composition lavante.

14. Composition lavante selon les revendications 12 et 13, laquelle composition lavante est une composition pour la peau, les cheveux, les ongles ou un objet inanimé.

15. Procédé de lavage, comprenant les étapes de :
a) hydratation de la composition nettoyante en poudre des revendications 1 à 11 pour produire une composition lavante ; et
b) mise en contact de la peau, des cheveux, des ongles ou d'un objet inanimé avec la composition lavante,
dans lequel la composition nettoyante en poudre est hydratée avec cisaillement pour produire une composition lavante avant ou après contact avec la peau, les cheveux, les ongles ou un objet inanimé.
